# EUROPEAN PATENT SPECIFICATION

(11) **EP 2 503 335 B1**
(45) Date of publication and mention of the grant of the patent: **07.01.2015**
(21) Application number: 10831774.4
(22) Date of filing: 16.11.2010
(51) Int. Cl.: G01N 33/53, G01N 33/52, B82B 3/00, G01N 33/543, B82Y 15/00

(54) **METHOD AND DEVICE FOR DETECTING ANALYTES**
VERFAHREN UND VORRICHTUNG ZUM NACHWEIS VON ANALYTEN
PROCÉDÉ ET DISPOSITIF POUR DÉTECTER DES SUBSTANCES À ANALYSER

(30) Priority: 17.11.2009 KR 20090110619
(43) Date of publication of application: 26.09.2012
(73) Proprietor: Amogreentech Co., Ltd., Kimpo-si, Kyonggi-do 415-863 (KR)
(72) Inventor: CHOI, Suk Jung, Gangneung-si Gangwon-do 210-140 (KR); CHOE, Byung Hak, Gangneung-si Gangwon-do 210-100 (KR); KIM, Sung Il, Gangneung-si Gangwon-do 210-101 (KR)
(74) Representative: Hano, Christian
(86) International application number: PCT/KR2010/008094
(87) International publication number: WO 2011/062407

(56) References cited:
- WO-A1-2009/062110
- WO-A1-2009/067595
- WO-A1-2009/126336
- WO-A2-2004/083902
- WO-A2-2005/003394
- WO-A2-2006/042089
- WO-A2-2007/065116
- WO-A2-2009/058853
- KARADIMITRIS, A. ET AL.: 'Human CD1d–glycolipid tetramers generated by in vitro oxidative refolding chromatography.' PROCEEDINGS OF THE NATIONAL ACADEMY OF SCIENCES vol. 98, no. 6, 13 March 2001, page 3294, 8211, 3298
- AMERSHAM BIOSCIENCES CO., LTD. &AUML KTAFPLC CATALOG December 2004, pages 1 - 16
- GRANT, A. G. ET AL.: 'Hybrid tetramers reveal elements of cooperativity in Escherichia coli D-3-phosphoglycerate dehydrogenase.' THE JOURNAL OF BIOLOGICAL CHEMISTRY vol. 278, no. 20, 16 May 2003, page 18170, 8211, 18176
- LAL, A. ET AL.: 'Clean western blot signals from immunoprecipitated samples.' MOLECULAR AND CELLULAR PROBES. vol. 19, no. 6, December 2005, page 385, 8211, 388
- AKBARI, O. ET AL.: 'Essential role of NKT cells producing IL-4 and IL-13 in the development of allergen-induced airway hyperreactivity.' NATURE MEDICINE. vol. 9, 31 March 2003, pages 582 - 588

## Description

The present invention relates to a method for detecting analytes, and more particular to a method for detecting analytes, in which an analyte-receptor complex that is formed by a coupling of an analyte and a receptor is separated from a free receptor that has not been coupled with the analyte, to then detect the analyte-receptor complex.

A biosensor is formed by fixing a receptor that acts as a sensing material to a signal transducer, and-has an advantage that can detect the receptor very sensitively through a specific and strong interaction between the receptor and the analyte. The receptor that is a substance that can be specifically coupled with the analyte, may be the antibody, DNA, carbohydrate, and the like, as a representative example. In such a biosensor, to detect different analytes, a different sensor chip immobilized with a receptor for each analyte must be used. Therefore, it costs much to develop the sensor chip and it is cumbersome to use the sensor chip.

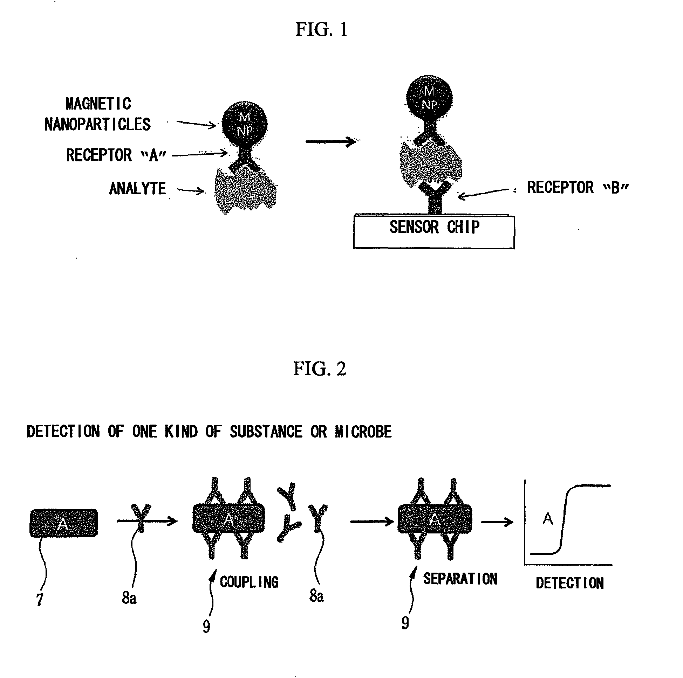

In addition, in the case of detecting an analyte mixed in a number of other substances such as food or blood, it has the disadvantage that it is difficult to accurately detect the analyte due to interference from other substances. To solve this problem, pretreatment methods of separating each analyte from other substances by using magnetic nanoparticles with a respectively immobilized receptor have been used. However, since particles that are not coupled with analytes as well as particles coupled with analytes are recollected together at the time of recovery of magnetic nanoparticles, another sensor chip with an immobilized receptor should be used in order to detect only the analyte (see FIG. 1).

WO 2009/067595 discloses a method of detecting analytes, for example a bacterium, wherein said method comprises combining the sample with a suspension of magnetic particles coupled with an analyte-specific antibody, separation and washing using a magnetic field holding the particles complexed with the analyte and releasing non-complexed molecules, transfer of the concentrated magnetic particles onto a filtration means and detection of the analyte.

WO 2007/065116 discloses a method to detect an analyte by capturing using fluorescent nanoparticles and then filtering, e.g. via a syringe.

WO 2004/083902 discloses fluorescent magnetic nanoparticles and separate them using magnetism or filtration.

WO 2005/003394 discloses a sandwich immunoassay in which an antibody is coupled to a magnetic particle. Another antibody is labelled with a detection probe (here a DNA marker for PCR), another particle (nanoparticle, mostly gold), and a fluorescent label. Separation can be done either using a magnetic field or filtration.

WO 2009/062110 discloses capturing an analyte using a fluorescent label, a magnetic label, or a nanoparticle. Mixing may be done using an external magnet. Before detection, the non-conjugated particles are filtered.

WO 2006/042089 discloses a method to detect antibodies in a sample by providing a receptor (a ligand) for the antibody. The complex aggregates, and is filtered.

WO 2009/126336 discloses a sandwich immunoassay, wherein the capture antibody is linked to a magnetic bead, and a signalling antibody, for example fluorescently labelled, is linked to a nanoparticle. Both antibodies bind to the analyte. A magnet is used to pull the complex and separate it from uncomplexed particles.

WO 2009/058853 discloses a method for detection of bacteria comprising first a step of concentrating the bacteria using an antibody attached to a magnetic particle, which may be a magnetic nanoparticle. The bacteria themselves are fluorescently labelled with viability dyes. Detection is performed by fluorescence.

To explain in more detail, assuming that a receptor immobilized with a magnetic nanoparticle is called "A," another receptor "B" should be immobilized in the sensor chip. The receptors "A" and "B" are coupled with different parts of the analyte. That is, coupling of one receptor should not affect coupling of the other receptors. Therefore, since two types of monoclonal antibodies are usually used for this detection method, a lot of efforts and costs are not only needed but also different sensor chips should be used for different analytes, respectively.

To solve the above problems or defects, it is an object of the present invention to provide a method and apparatus for detecting analytes in which an analyte-receptor complex that is formed by a coupling of an analyte and a receptor is separated from a free receptor that has not been coupled with the analyte, using a micro-filter for filtering the analyte-receptor complex and passing the free receptor that has not been coupled with the analyte, to then detect the analyte-receptor complex.

It is another object of the present invention to provide a method and apparatus for detecting analytes in which various types of analytes can be detected by one type of sensor chips immobilized with one type of receptors by adding a separation function to a biosensor.

It is still another object of the present invention to provide a method for detecting analytes in which an analyte-receptor complex that is formed by a coupling of an analyte and a receptor is separated from a free receptor that has not been coupled with the analyte, in a tube having a selective filter, to then directly detect the analyte-receptor complex in the tube without using a sensor chip and to thereby enhance convenience and sensitivity.

It is yet another object of the present invention to provide a method for detecting analytes in which pretreatment using magnetic nanoparticles and detection of analytes are accomplished in a tube to thereby provide an effect of high convenience and economy.

To accomplish the above and other objects of the present invention, according to the present invention, there is provided a method of detecting analytes, comprising the steps of:
putting receptors into a sample containing the analytes to thus induce the receptors and the analytes to be coupled with each other, to thereby form analyte-receptor complexes that are obtained by a coupling of the receptors with the analytes, respectively;
separating the analyte-receptor complexes from free receptors that have not been coupled with the analytes; and
detecting the analyte-receptor complexes separated from the free receptors.

According to the present invention, there is provided a method of detecting analytes, comprising the steps of:
putting a sample containing analytes and fluorescent-magnetic nanoparticles immobilized with receptors into a tube attached with a micro-filter, to thus induce to couple the analytes with the nanoparticles, respectively;
making the fluorescent-magnetic nanoparticles attached to the wall of the tube at a state where a magnetic field is applied by a magnet that is placed on the outer portion of the tube;
applying a suction force to the remaining sample except for the fluorescent-magnetic nanoparticles attached to the tube wall to thus remove the remaining sample from the tube without passing through the micro-filter;
putting a predetermined amount of a buffer solution into the tube and applying the suction force to the buffer solution, to thus remove the buffer solution from the tube without passing through the micro-filter, and to thereby remove impurities that can affect measurement of fluorescence;
putting a predetermined amount of a buffer solution into the tube at a state where the magnetic field by the magnet has been removed, and
applying the suction force from the outer portion of the micro-filter, to thereby remove the free nanoparticles through the micro-filter; and
measuring fluorescence emitted from the coupled nanoparticles remaining in the micro-filer, to thus detect the analytes.

As described above, according to the present invention, an analyte-receptor complex that is formed by a coupling of an analyte and a receptor is separated from free receptors that have not been coupled with analytes, to then detect the analyte-receptor complex, and to thereby obtain the same effect as that of directly detecting the analytes.

In the case of detecting the analyte-receptor complex as described above, various types of analytes can be detected by one type of sensor chips immobilized with one type of receptors by adding a separation function to a biosensor, differently from the case of directly detecting the analytes.

For example, when various types of analytes are detected, an antibody with respect to each analyte is produced in a goat and is used as a receptor. In this case, various types of analyte-antibody complexes are all detected with a sensor chip immobilized with a secondary antibody with respect to the goat antibody, to thereby obtain an effect of improving convenience and affordability.

In addition, according to the present invention, an analyte-receptor complex that is formed by a coupling of an analyte and a receptor is separated from free receptors that have not been coupled with analytes, in a tube equipped with a selective filter, to then directly detect the analyte-receptor complex, without using a sensor chip, and to thereby obtain an effect of improving convenience and sensitivity.

Furthermore, according to the present invention, pretreatment and detection of analytes using magnetic particles, are accomplished in a single tube, to thereby obtain an effect of enhancing convenience and economy.
FIG. 1 is a diagram for explaining a conventional method of separating an analyte with a magnetic nanoparticle immobilized with a first receptor to thus produce an analyte-receptor complex, and detecting the analyte-receptor complex by using a sensor chip coupled with a second receptor that recognizes different parts from that of the first receptor, to thereby detect the analytes.

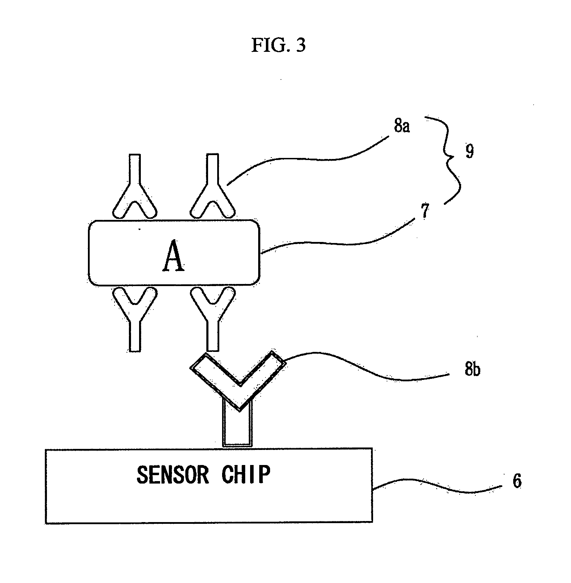

FIG. 2 is a diagram for explaining a method of separating an analyte-receptor complex from free receptors that have not been coupled with analytes to then detect the analyte-receptor complex, according to the present invention, and FIG. 3 is a diagram for explaining a case of detecting the analyte-receptor complex by using a sensor chip equipped with a secondary receptor with respect to the receptor.

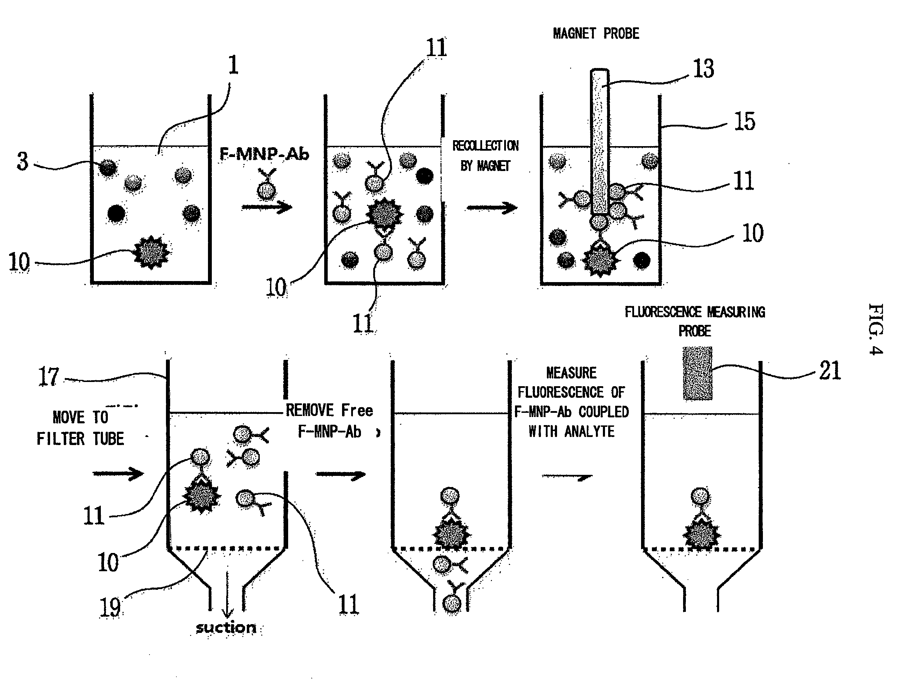

FIG. 4 is a diagram for explaining a method of detecting bacteria by performing a pretreatment of coupling a fluorescent magnetic nanoparticle immobilized with an antibody (hereinafter referred to as a fluorescent magnetic nanoparticle-antibody; F-MAP-Ab) with bacteria in a reaction cup and recollecting the fluorescent magnetic nanoparticle-antibody (F-MAP-Ab), to then remove free fluorescent magnetic nanoparticle-antibodies (F-MAP-Abs) that have not been coupled with bacteria in a filter tube, and measure fluorescence.

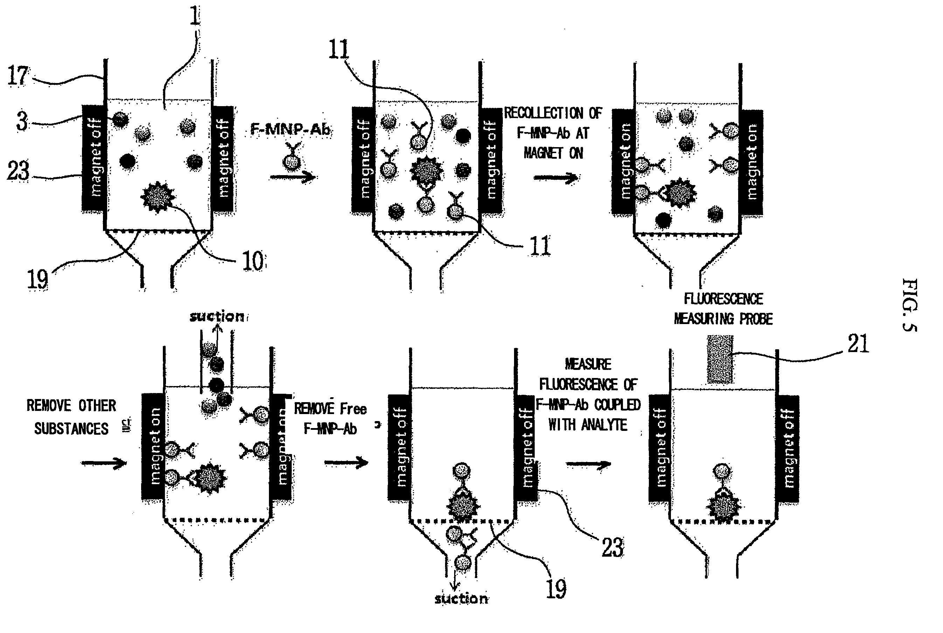

FIG. 5 is a diagram for explaining a method of detecting bacteria by performing a pretreatment using the fluorescent magnetic nanoparticle-antibody (F-MAP-Ab) in a filter tube, to then remove free fluorescent magnetic nanoparticle-antibodies (F-MAP-Abs) and measure fluorescence, according to the present invention.

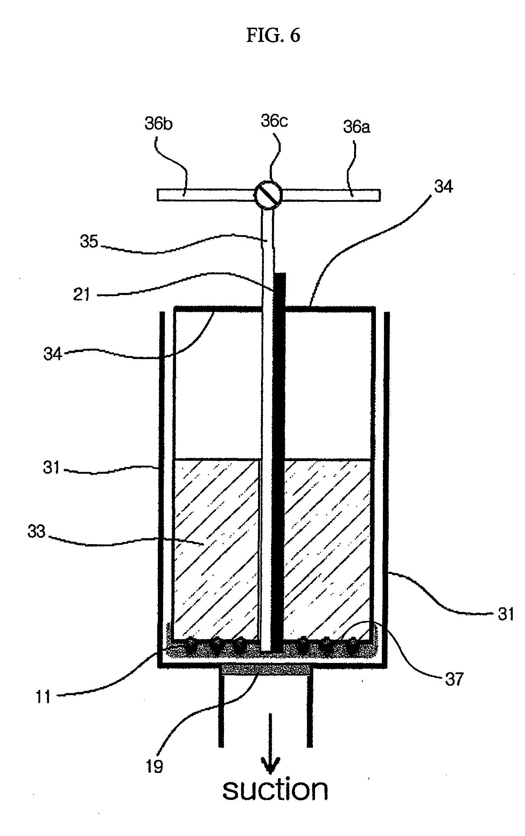

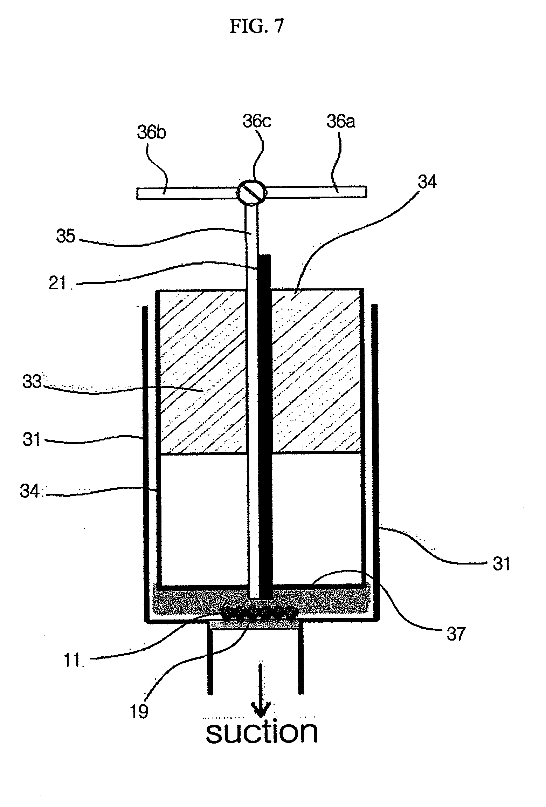

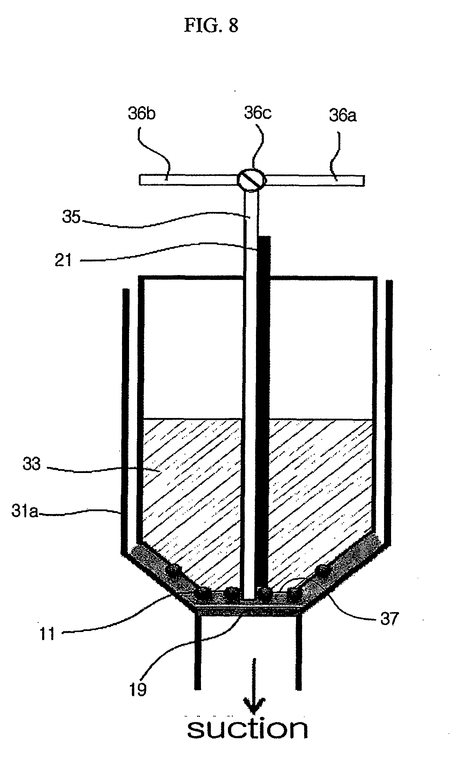

FIGS. 6 and 7 are diagrams for explaining a method of detecting bacteria by performing a pretreatment using the fluorescent magnetic nanoparticle-antibody (F-MAP-Ab) in a filter tube having a magnet to which a penetration tube and a fluorescence measuring probe are attached, to then remove free F-MAP-Abs and measure fluorescence, respectively, and FIG. 8 is a cross-sectional view showing a variation of an analyte separating device.

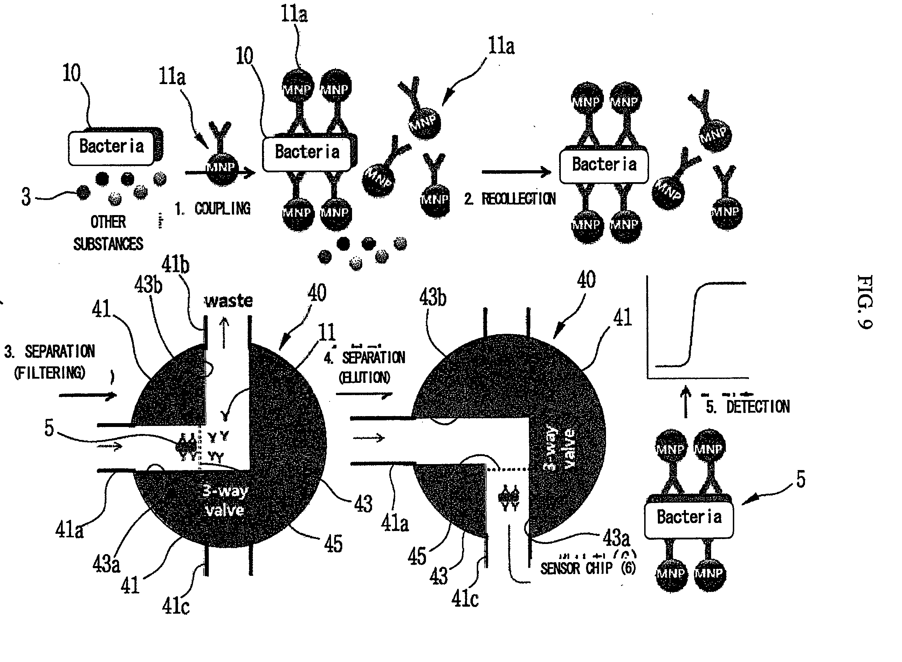

FIG. 9 is a diagram for explaining a method of detecting analytes by filtering a bacteria-antibody complex with a filter attached to a three-way valve, and changing the direction of flow to thus extract the bacteria-antibody complex, to then be injected into a sensor chip.

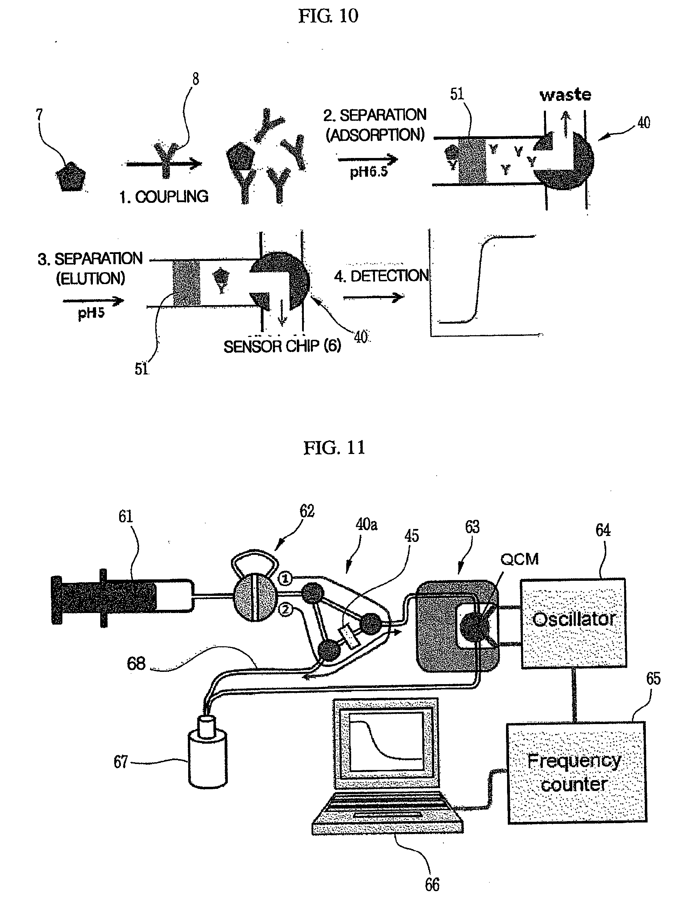

FIG. 10 is a diagram for explaining a method of separating an analyte-antibody complex from free antibodies by using an anion-exchange filter and a 3-way valve and detecting the separated analyte-antibody complex by using a sensor chip.
FIG. 11 is a schematic diagram showing a bio-sensor system to which a micro-filter and a 3-way valve that can change the direction of flow are attached in accordance with the present invention.

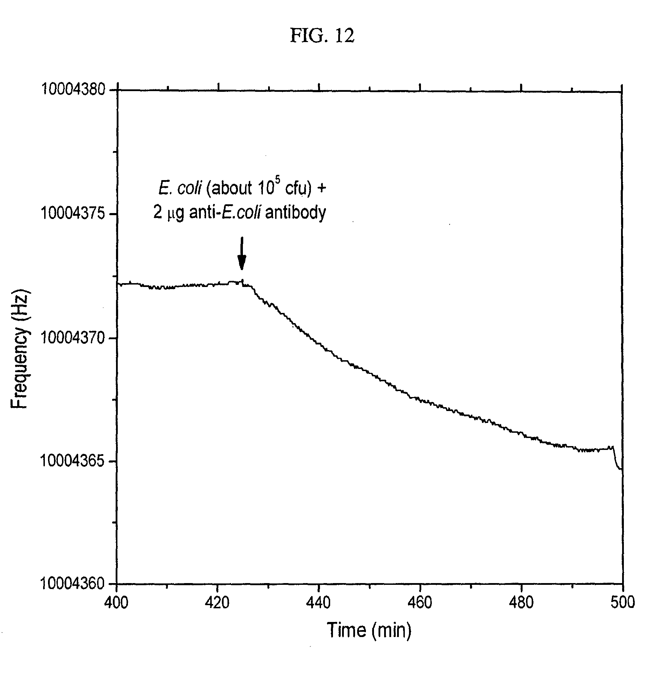

FIG. 12 is a graphical view illustrating experimental results of experiments of separating bacteria-antibody complexes from free antibodies that have not been coupled with bacteria to then detect the bacteria-antibody complexes, in the system of FIG. 11.

Hereinafter, specific methods according to the present invention will be described in detail according to embodiments of the present invention.

A method of detecting analytes according to the present invention, includes the steps of: putting receptors into a sample containing the analytes to thus induce the receptors and the analytes to be coupled with each other, and to thereby form analyte-receptor complexes (hereinafter referred to as "complexes") that are obtained by coupling the receptors with the analytes, respectively; separating the complexes from receptors that are not coupled with the analytes (hereinafter referred to as "free receptors"); and detecting the complexes separated from the free receptors.

Here, the analytes are substances that become detection targets. There are bacteria, viruses, proteins, nucleic acids, carbohydrates, lipids, metal ions, organic compounds and the like, as typical examples of the analytes.

In addition, the receptors are substances that are specifically coupled with the analytes, respectively. There are antibodies or proteins including enzymes, peptides, nucleic acids, carbohydrates, and the like, as typical examples of the receptors.

FIG. 2 is a diagram for explaining a method of separating a complex from free receptors to then detect the complex, according to the present invention, and FIG. 3 is a diagram for explaining a case of detecting the complex by using a sensor chip equipped with a secondary receptor with respect to the receptor.

Referring to FIGS. 2 and 3, the analyte detection method according to the present invention, includes the steps of: adding receptors 8a to a first analyte (A) 7 to thus induce to couple the analyte (A) 7 and the receptors 8a; separating an analyte-receptor complex 9 from free receptors 8a and then detecting the separated complex 9 by using a sensor chip 6 immobilized with a secondary receptor 8b with respect to the receptor 8a of the complex 9.

Since the complex 9 and the free receptors 8a differ from each other in view of various characteristics such as sizes, charges, and isoelectric points, this inventive method can separate the complex 9 and the free receptors 8a through a selective filter by using the different characteristics thereof.

The selective filter represents a filter that selectively filters or adsorb only a complex to thus separate the complex from the free receptors. There may be micro-filters or ion-exchange filters as typical examples of the selective filters.

Since bacteria are several micrometers in size, whereas antibodies are 10 nanometers in size in the case of detecting bacteria that are analytes by using antibodies as receptors, respectively, it is possible to selectively filter only bacteria-antibody complexes by using a micro-filter having a hole of about 0.2 to 1 micrometer.

The thus-separated bacteria-antibody complexes can be advantageously detected by any method of detecting antibodies. For example, bacteria-antibody complexes can be detected by a sensor chip immobilized with a secondary antibody. Here, the secondary antibody indicates an antibody for immunoglobulin G protein of the goat when the antibodies that are coupled with bacteria were made in the goat. Thus, if antibodies generated in the goat are used for various types of bacteria, all the bacteria-antibody complexes can be detected by using a sensor chip immobilized with the secondary antibody with respect to the goat immunoglobulin G.

In other words, in the case that the analytes 7 are bacteria and the receptors 8a are antibodies, the sensor chip 6 immobilized with a secondary antibody as a secondary receptor 8b is used to detect bacteria-antibody complexes in which the bacteria and the antibodies are coupled with each other.

In addition, any sensor chips having a surface that can be respectively coupled with antibodies can be used. For example, protein such as protein A or protein G may be used.

Besides, if appropriate markers are respectively attached on antibodies in advance, it is also possible to use a simpler detection method. For example, in the case of samples containing many substances as in food and serum, there is a need to separate bacteria using magnetic nanoparticles. Accordingly, the same method as that of FIG. 4 can be used by introducing fluorescence on the magnetic nanoparticles. Of course, the markers introduced on the magnetic nanoparticles are not limited to only the fluorescence but any markers that enable high-sensitivity detection such as radioactive rays, emission, or enzyme can be applied.

FIG. 4 is a diagram for explaining a method of detecting bacteria by performing a pretreatment of coupling a fluorescent magnetic nanoparticle immobilized with an antibody (hereinafter referred to as a fluorescent magnetic nanoparticle-antibody; F-MAP-Ab) with bacteria in a reaction cup and recollecting the fluorescent magnetic nanoparticle-antibody (F-MAP-Ab), to then remove free fluorescent magnetic nanoparticle-antibodies (F-MAP-Abs) that have not been coupled with bacteria in a filter tube, and measure fluorescence.

In the case of the analyte detection method as shown in FIG. 4, fluorescent magnetic nanoparticle-antibodies (F-MNP-Abs) 11 are put into a sample 1 in a single reaction cup 15 so as to be coupled with bacteria 10. Then, if a recollection is performed by using a magnet probe 13, the F-MNP-Abs 11 that have been coupled with bacteria 10 and the free F-MNP-Abs 11 that have not been coupled with bacteria 10 are all recollected. In FIG. 4, a reference numeral 3 denotes substances other than the analytes.

These F-MNP-Abs 11 and the free F-MNP-Abs 11 are moved to a filter tube 17 on the bottom of which a micro-filter 19 having a hole of about 0.2 to 1 micrometer is attached. The free F-MNP-Abs 11 are removed by applying a suction force to the lower end of the tube 17. The free F-MNP-Abs 11 are completely removed by adding a suitable buffer solution and applying a suction force again. Finally, after adding an appropriate buffer solution, fluorescence emitted from the F-MNP-Abs remaining at a state of being coupled with bacteria in the micro-filter 19 is measured by using a fluorescence measuring probe 21, to thereby determine the analytes.

Unless the sample is too turbid, it is also possible to perform the whole process in the single tube as shown in FIG. 5.

FIG. 5 is a diagram for explaining a method of detecting bacteria by performing a pretreatment using the fluorescent magnetic nanoparticle-antibody (F-MAP-Ab) in a filter tube, to then remove free fluorescent magnetic nanoparticle-antibodies (F-MAP-Abs) and measure fluorescence, according to the present invention.

In the case of the analyte detection method according to the the present invention, as shown in FIG. 5, a sample 1 is firstly put into a filter tube 17. Here, fluorescent magnetic nanoparticle-antibodies (F-MNP-Abs) 11 are put into the sample 1 so as to be coupled with bacteria 10. Then, the F-MNP-Abs 11 are recollected by applying a magnetic field of a magnet 23 surrounding the walls of the tube 17 to the F-MNP-Abs 11. If an electromagnet is used as the magnet surrounding the tube walls, electricity is applied to coil of the electromagnet so as to apply the magnetic field to the F-MNP-Abs 11. Meanwhile, if a permanent magnet is used as the magnet surrounding the tube walls, the permanent magnet is made to move and contact the tube, to thus apply the magnetic field to the F-MNP-Abs 11.

Thereafter, a solution that contains the other substances 3 is removed through a suction force from the upper part of the tube. Then, a buffer solution is added and a suction force is applied again from the upper part of the tube so as to be washed. Then, the magnetic field of the magnet 23 is released and a suction force is applied from the lower end of the tube, to thus remove the free F-MNP-Abs 11. The free F-MNP-Abs 11 are completely removed by adding a suitable buffer solution and applying a suction force again. Finally, after adding an appropriate buffer solution, fluorescence emitted from the F-MNP-Abs remaining at a state of being coupled with bacteria in the micro-filter 19 is measured by using the fluorescence measuring probe 21, to thereby determine the analytes.

In addition, since it can take a long time to recollect the F-MNP-Abs 11 when the magnet 23 surrounding the walls of the tube 17 is used as in the present invention, devices shown in FIGS. 6 to 8 can be used in order to shorten the recovery time and to simplify the process.

FIGS. 6 and 7 are diagrams for explaining a method of detecting bacteria by performing a pretreatment using the fluorescent magnetic nanoparticle-antibody (F-MAP-Ab) in a filter tube having a magnet to which a penetration tube and a fluorescence measuring probe are attached, to then remove free F-MAP-Abs and measure fluorescence, respectively, and FIG. 8 is a cross-sectional view showing a variation of an analyte separating device.

The analyte detection method uses respective analyte detection apparatuses shown in FIGS. 6 to 8.

The analyte detection apparatus is equipped with a micro-filter 19 the bottom of the tube 31, in which the micro-filter 19 filters the F-MNP-Abs 11 that have been coupled with the analytes and passes free F-MNP-Abs 11 that have not been coupled with the analytes.

In addition, a magnet 33 built in a magnet housing 34 is placed in the inside of the tube 31, so as to quickly recollect the F-MNP-Abs 11.

A penetration tube 35 and a fluorescence measuring probe 21 are inserted into a throughhole located at the center of the magnet 33. Two tubes 36a and 36b are connected to the penetration tube 35, via a 3-way valve 36c, in which one of the two tubes is used to inhale the solution in the tube, and the other is used to supply the buffer solution. By switching the direction of the valve 36c, the penetration tube 35 inserted into the magnet 33 is connected with a buffer solution supply tube 36a or a suction tube 36b.

An electromagnet or a permanent magnet may be used as the magnet 33. When a permanent magnet is used as the magnet 33, the permanent magnet is built in a magnet housing 34 so as to move up and down, and a separator 37 is provided on the bottom of the magnet housing 34, as shown in FIG. 7. Accordingly, when the magnet is moved down, the F-MNP-Abs 11 are recollected through the magnetic force, while when the magnet is moved up, the F-MNP-Abs 11 are discharged from the separator 37. Here, the example of using the permanent magnet will be described.

In addition, the analyte detection apparatus as shown in FIG. 8 employs a tube 31a and a magnet 33 each of which diameter is gradually reduced from the lower parts of the tube 31 a and magnet 33 to the bottom of a magnet housing on the bottom of which a micro-filter 19 is placed, to thereby broaden a contact area between the F-MNP-Abs 11 and the magnet 33 and to thus make it easy for the solution to flow down through the lower end of the tube as well as to thus recollect the F-MNP-Abs 11 more quickly.

In the case of the analyte detection method according to the present invention, a sample 1 and F-MNP-Abs 11 are put into a filter tube 31, in order to couple F-MNP-Abs 11 and bacteria 10, and then the F-MNP-Abs 11 are recollected by the magnetic force by lowering the permanent magnet 33 down to part of the separator 37, as shown in FIG. 6. At this stage, it is recommended that the magnet housing 34 including the magnet is positioned close to the floor of the filter tube 31 at maximum in order to reduce the time required for recovery.

Then, the 3-way valve 36c is manipulated so that the penetration tube 35 is connected to the suction tube 36b, to thus inhale the sample 1 and to thereby remove a solution containing the other substances 3. Thus, only the F-MNP-Abs 11 adsorbed on the lower surface of the separator 37 remain in the inside of the filter tube 31.

Then, the 3-way valve 36c is manipulated so that the penetration tube 35 is connected to the buffer solution supply tube 36a and then the buffer solution is added through the buffer solution supply tube 36a into the filter tube 31, to then inhale the buffer solution with the suction tube 36b again, to thereby wash the F-MNP-Abs 11. In this step, it is desirable that the magnet housing 34 should be raised up a little in order to perform the washing process smoothly.

Subsequently, as shown in FIG. 7, when the permanent magnet 33 is moved up within the magnet housing 34, the F-MNP-Abs 11 are separated from the separator 37. Thereafter, The free F-MNP-Abs 11 that have not been coupled with the bacteria 10 are removed by applying a suction force to the lower end of the micro-filter 19. Then, the free F-MNP-Abs 11 are completely removed by adding a suitable buffer solution again and applying a suction force again to the lower end of the micro-filter 19. Finally, after adding an appropriate buffer solution, fluorescence emitted from the F-MNP-Abs 11 remaining at a state of being coupled with the bacteria 10 in the micro-filter 19 is measured by using a fluorescence measuring probe 21, to thereby determine the analytes.

The methods of using the filter tubes as described above are very simple, to thus make it easier to automate the processes, as well as to become very highly economic, if only a F-MNP-Ab is developed for each of bacteria to be detected.

In the case that more accurate measurements are needed, as shown in FIG. 9, a 3-way valve is used to thus separate bacteria-antibody complexes and then detect the separated bacteria-antibody complexes with the sensor chip.

FIG. 9 is a diagram for explaining a method of detecting analytes by filtering a bacteria-antibody complex with a filter attached to a three-way valve, and changing the direction of flow to thus extract the bacteria-antibody complex, to then be injected into a sensor chip.

The analyte detection apparatus: a 3-way valve 40 that separates bacteria-antibody complexes 5 that have been coupled with bacteria and free antibodies that have not been coupled with the bacteria; and a bio-sensor chip 6 immobilized with a secondary antibody so as to detect the bacteria-antibody complexes 5.

The 3-way valve 40 includes: a housing 41 having a first port 41 a through which a sample containing the bacteria-antibody complexes 5 and the free receptors that have not been coupled with the bacteria is supplied, a second port 41 b through which the free receptors are discharged, and a third port 41 c through which the bacteria-antibody complexes 5 are discharged to the bio-sensor chip; a rotating body 43 that is rotatably provided in the housing 41, and includes an internal passageway that is connected to first and second inlets 43a and 43b, in which the first and second inlets 43a and 43b are respectively matched to the first and second ports 41 a and 41 b at an initial state and the first and second inlets 41 a and 41 b are respectively matched to the third and first ports 41 c and 41 a at a rotating state; and a micro-filter 45 that is provided in the internal passageway between the first and second inlets 43a and 43b of the rotating body 43 and filters the bacteria-antibody complexes 5 and passes the free receptors.

Referring to FIG. 9, in the case of the analyte detection method, particles (hereinafter referred to as "MNP-Abs") 11a that are formed by coupling magnetic nanoparticles (MNPs) with antibodies, respectively, are firstly put into a sample that contains bacteria 10 and other substances 3 to thereby induce the bacteria 10 and the MNP-Abs 11a and to thus produce bacteria-MNP-Ab complexes 5.

Subsequently, the bacteria-MNP-Ab complexes 5 and the MNP-Abs 11 a that are not coupled with the bacteria 10 (hereinafter referred to as "free MNP-Abs") are separated and recollected from the sample by using the magnet according to the above-mentioned method.

Subsequently, the bacteria-MNP-Ab complexes 5 and the free MNP-Abs 11 a are put into the first port 41 a of the 3-way valve 40, to thereby filter the bacteria-MNP-Ab complexes 5 through a micro-filter 45 and discharge the free MNP-Abs 11a through a second port 41 b.

Subsequently, when a buffer solution is supplied by rotating the rotating body 43 of the 3-way valve 40 to change the direction of flow, only bacteria-MNP-Ab complexes 5 can be obtained to then be injected into the sensor chip 6. In this case, the sensor chip 6 is immobilized with the secondary antibodies to thus trap the bacteria-MNP-Ab complexes 5.

Here, it is noted that it is possible to use a sensor chip immobilized not with the antibodies for the bacteria but with the secondary antibodies since the free MNP-Abs 11 a that are not coupled with the bacteria 10 have been already removed, and only the bacteria-MNP-Ab complexes 5 remain. As a result, all kinds of bacteria can be detected by the sensor chip 6 to thus increase the convenience and affordability.

Detection of the bacteria-MNP-Ab complexes 5 can be achieved in SPR biosensors using a surface plasmon resonance (SPR) phenomenon and sensor chips employing all kinds of methods such as quartz crystal microbalance (QCM) using a piezoelectric phenomenon. Furthermore, detection of the bacteria-MNP-Ab complexes 5 may be done even by sensor chips using giant magnetoresistance (GMR) since bacteria is immobilized to magnetic nanoparticles (MNPs). In addition, if fluorescence properties are given to magnetic nanoparticles (MNPs) or enzymes causing color reaction are connected with the magnetic nanoparticles (MNPs), detection of the bacteria-MNP-Ab complexes 5 may be done even by sensor chips employing methods of measuring fluorescence or absorbance. Even in the case that antibodies are not immobilized with magnetic nanoparticles (MNPs), the same sensor chips as the above-described ones can be used by giving fluorescence properties to magnetic nanoparticles (MNPs) or connecting enzymes causing color reaction with the magnetic nanoparticles (MNPs).

In this way, various kinds of differences in characteristics such as sizes, charges, and isoelectric points may be used in the case that of detecting small substances such as protein, but a method of using the isoelectric points will be described here, for example.

The isoelectric points means the hydrogen-ion concentration index (pH) that the net charge of a particular protein becomes zero. If the pH becomes higher than the isoelectric point, the protein has a negative net charge and if the former becomes lower than the latter, the protein has a positive net charge. If the isoelectric point of the analyte is 5 and the isoelectric point of the antibody is 7, the isoelectric point of the analyte-antibody complexes is greater than 5 and less than, for example, a value of around 6. Thus, it is possible to find a pH condition under which free antibodies that have not been coupled with analytes are not coupled with an anion-exchange filter but only the analyte-antibody complexes are coupled therewith.

FIG. 10 is a diagram for explaining a method of separating an analyte-antibody complex from free antibodies by using an anion-exchange filter and a 3-way valve and detecting the separated analyte-antibody complex by using a sensor chip.

For example, if a condition under which free antibodies are not coupled but only analyte-antibody complexes are coupled is satisfied at pH6.5, antibodies 8 are firstly put into a sample that contains analytes 7 to thus induce to couple the analytes 7 with the antibodies 8, and to then pass through the anion-exchange filter 51 in the buffer solution of pH6.5. Accordingly, only the analyte-antibody complexes are adsorbed by the anion-exchange filter 51 and the free antibodies 8 that have not been coupled with the analytes 7 are discharged through an outlet in a waste direction through the 3-way valve 40.

Subsequently, if a buffer solution whose pH has been adjusted into 5 is made to flow at a state where the direction of the outlet of the 3-way valve 40 is diverted into the direction of the sensor chip 6, analyte-antibody complexes are erupted in the anion-exchange filter 51 to then be discharged through the 3-way valve 40 to the sensor chip 6. The separated analyte-antibody complexes can be detected in the same manner as the detection method of the previously described bacteria-antibody complexes. Further, as the MNP-Abs can be also applied in the same manner as that of the fifth embodiment, it is possible to perform high-sensitivity detection using pretreatment and magnetic nanoparticles.

Besides, when the selective filter, that is, the micro-filter 19 is changed into an ion-exchange filter in the methods using the filter tube shown in FIGS. 4 and 5 and the pH of the buffer solution is changed, it is possible to adsorb only F-MNP-Abs 11 that are coupled with the analytes 7 to the ion-exchange filter and remove the free F-MNP-Abs 11 that have not been coupled with the analytes.

If only selective filters that discriminate analyte-receptor complexes from free receptors that have not been coupled with analytes, to thereby selectively filter or adsorb the analyte-receptor complexes and the free receptors are provided, the above-described methods can also be applied to the analytes such as bacteria, proteins, nucleic acids, carbohydrates, and organic substances, and heavy metals.

The preferred example will be described below in greater detail.

### <Example 1> Only antibody-bacteria complexes are separated to thus detect bacteria.

In order to determine how to separate antibody-bacteria complexes from antibodies that have not been coupled with bacteria, and then detect the separated antibody-bacteria complexes, a biosensor system using QCM shown in FIG. 11 has been made.

FIG. 11 is a schematic diagram showing a bio-sensor system to which a micro-filter and a 3-way valve that can change the direction of flow are attached.

The bio-sensor system shown in FIG. 11 includes: a pump 61 that pumps a buffer solution to flow; an injection valve 62 for injecting Escherichia (E.) coli antibodies and colon bacilli; a 3-way valve 40a; a cell 63 accommodating a QCM sensor chip immobilized with a secondary antibody for a goat immunoglobulin G protein; and a waste collection bottle 67 that are sequentially connected through conduit 68. An oscillator 64, a frequency counter 65 and a detection signal analysis computer 66 are connected in sequence with the QCM sensor chip.

According to the characteristics of this system, a micro-filter 45 having holes with 0.5µm or so in size that can filter antibody-bacteria complexes is provided between the injection valve 62 and the cell 63 of the QCM sensor chip, to thus change the direction of flow into directions of ① and ② by using the 3-way valve 40a.

In this experiment, a phosphate buffer solution (PBS) was used as a carrying buffer solution, and a flow rate was set as 50µℓ/min. The QCM sensor chip was immobilized with the secondary antibody for goat immunoglobulin G protein.

First, the anti-Escherichia (E.) coli antibody raised in the goat of 10µg was injected and then the direction of flow of the 3-way valve 40a was changed into ①. Then, the buffer solution was made to flow for 30 minutes. Then, the direction of flow of the 3-way valve 40a was changed into ②, to then have observed change in frequency. As a result, there was no change in frequency. This showed that the QCM sensor chip in the cell did not detect any antibodies for the E. coli, since antibody protein passed through the filter and were removed in the direction of flow of ①.

Then, after having mixed antibodies of 10µg against E. coli with 10⁵ cfu of E. coli and having injected the mixture, the same experiment as the above-described experiments was preformed. As a result, as shown in FIG. 12, it was confirmed that frequency decreases. In other words, since antibodies that have been coupled with E. coli did not pass through the filter, they were trapped by the filter. Then, when the direction of flow is changed into to the direction of ②, the coupled antibodies entered the cell 63 of the QCM sensor chip to thus be detected in the sensor chip.

The above results show that antibodies that have been coupled with bacteria and antibodies that have not been coupled with bacteria are separated by using the filter with suitable holes to then detect bacteria-antibody complexes.

As described above, the present invention adds a separation function to a biosensor, to thereby detect various types of analytes with a sensor chip.

The present invention can be applied for an apparatus that detect analytes such as bacteria, protein, nucleic acids, organic compounds, and heavy metals. As described above, the present invention has been described with respect to particularly preferred embodiments.

## Claims

1. A method of detecting analytes (10), comprising the steps of:
putting receptors into a sample (1) containing the analytes (10) to thus induce the receptors and the analytes (10) to be coupled with each other, and to thereby form analyte-receptor complexes that are obtained by a coupling of the receptors with the analytes (10), respectively;
separating the analyte-receptor complexes from free receptors that have not been coupled with the analytes (10); and wherein the receptors are used at a state being immobilized with fluorescent-magnetic nanoparticles (11); and
detecting the analyte-receptor complexes separated from the free receptors;
**characterized in that** the step of separating the analyte-receptor complexes comprises the steps of:
(a) putting a sample (1) containing analytes (10) and fluorescent-magnetic nanoparticles (11) immobilized with the receptors into a tube (17) attached with a selective filter (19),
(b) making the fluorescent-magnetic nanoparticles (11) attached to the wall of the tube (17) at a state where a magnetic field is applied by a magnet (23) that is placed on the outer portion of the tube (17),
(c) applying a suction force to thus remove the remaining sample (1) except for the fluorescent-magnetic nanoparticles (11) attached to the tube wall from the tube (17),
(d) putting a predetermined amount of a buffer solution into the tube (17) at a state where the magnetic field by the magnet (23) has been removed,
(e) and applying the suction force from the outer portion of the selective filter (19) that filters the fluorescent-magnetic nanoparticles (11) that have been coupled with the analytes (10) and
passes the free fluorescent-magnetic nanoparticles (11) that have not been coupled with the analytes (10),
to then pass through the selective filter (19) and to thereby remove the free fluorescent-magnetic nanoparticles (11) that have not been coupled with the analytes (10).

2. The method of claim 1, wherein the analytes (10) are detected by measuring fluorescence emitted from the fluorescent-magnetic nanoparticles (11) remaining in the filter (19).

## Patentansprüche

1. Verfahren zum Nachweis von Analyten (10) mit den Schritten:
Geben von Rezeptoren in eine die Analyten (10) enthaltende Probe (1), um so ein Koppeln der Rezeptoren und der Analyten (10) miteinander zu bewirken und dadurch Analyt-Rezeptor-Komplexe zu bilden, die jeweils durch ein Koppeln der Rezeptoren mit den Analyten (10) erhalten werden;
Trennen der Analyt-Rezeptor-Komplexe von den freien Rezeptoren, die nicht mit den Analyten (10) gekoppelt wurden, wobei die Rezeptoren in einem Zustand verwendet werden, in dem sie mit fluoreszent-magnetischen Nanopartikeln (11) immobilisiert wurden; und
Nachweisen der Analyt-Rezeptor-Komplexe getrennt von den freien Rezeptoren;
**dadurch gekennzeichnet, dass** der Schritt des Trennens der Analyt-Rezeptor-Komplexe die Schritte aufweist:
(a) Geben einer Probe (1), die Analyten (10) und mit den Rezeptoren immobilisierte fluoreszent-magnetische Nanopartikel (11) enthält, in ein Röhrchen (17), an dem ein selektiver Filter (19) befestigt ist,
(b) Veranlassen, dass die fluoreszent-magnetischen Nanopartikel (11) an der Wand des Röhrchens (17) in einem Zustand angeheftet werden, in dem ein Magnetfeld durch einen Magneten (23) angelegt wird, der an dem Außenabschnitt des Röhrchens (17) positioniert ist,
(c) Anlegen einer Saugkraft, um dadurch die restliche Probe (1) außer den an der Röhrchenwand des Röhrchens (17) angehefteten, fluoreszent-magnetischen Nanopartikeln (11) zu entfernen,
(d) Geben einer vorbestimmten Menge einer Pufferlösung in das Röhrchen (17) in einem Zustand, in dem das Magnetfeld des Magneten (23) entfernt wurde,
(e) und Anlegen der Saugkraft von dem Außenabschnitt des selektiven Filters (19), der die fluoreszent-magnetischen Nanopartikel (11) filtert, die mit den Analyten (10) gekoppelt wurden, und
die freien fluoreszent-magnetischen Nanopartikel (11) durchlässt, die nicht mit den Analyten (10) gekoppelt wurden,
um dann durch den selektiven Filter (19) hindurchzugehen und dadurch die nicht mit den Analyten (10) gekoppelten freien fluoreszent-magnetischen Nanopartikel (11) zu entfernen.

2. Verfahren nach Anspruch 1, bei welchem die Analyten (10) durch Messen der Fluoreszenz, die von den in dem Filter (19) verbleibenden fluoreszent-magnetischen Nanopartikeln (11) emittiert wird, nachgewiesen werden.

## Revendications

1. Procédé de détection d'analytes (10), comprenant les étapes consistant à :
placer des récepteurs dans un échantillon (1) contenant les analytes (10) afin d'induire le couplage des récepteurs et des analytes (10) et de former ainsi des complexes analytes-récepteurs qui sont obtenus par un couplage des récepteurs avec les analytes (10), respectivement ;
séparer les complexes analytes-récepteurs des récepteurs libres qui n'ont pas été couplés aux analytes (10) ; et les récepteurs étant utilisés dans des conditions d'immobilisation par des nanoparticules magnétiques fluorescentes (11) ; et
détecter les complexes analytes-récepteurs séparés des récepteurs libres ;
**caractérisé en ce que** l'étape de séparation des complexes analytes-récepteurs comprend les étapes consistant à :
(a) placer dans un tube (17) muni d'un filtre sélectif (19) un échantillon (1) contenant des analytes (10) et des nanoparticules magnétiques fluorescentes (11) immobilisées par les récepteurs,
(b) faire en sorte que les nanoparticules magnétiques fluorescentes (11) collent à la paroi du tube (17) dans des conditions d'application d'un champ magnétique par un aimant (23) qui est placé sur la partie extérieure du tube (17),
(c) appliquer une force d'aspiration pour retirer du tube (17) le reste d'échantillon (1) à l'exception des nanoparticules magnétiques fluorescentes (11) collées à la paroi du tube,
(d) verser dans le tube (17) une quantité prédéfinie d'une solution tampon dans des conditions d'absence d'application du champ magnétique par l'aimant (23),
(e) et appliquer la force d'aspiration depuis l'extérieur du filtre sélectif (19), qui filtre les nanoparticules magnétiques fluorescentes (11) qui ont été couplées aux analytes (10) et laisse passer les nanoparticules magnétiques fluorescentes (11) libres qui n'ont pas été couplées aux analytes (10), pour que, passant à travers le filtre sélectif (19), les nanoparticules magnétiques fluorescentes (11) libres, qui n'ont pas été couplées aux analytes (10), soient éliminées.

2. Procédé selon la revendication 1, dans lequel les analytes (10) sont détectés en mesurant la fluorescence émise par les nanoparticules magnétiques fluorescentes (11) restant dans le filtre (19).
